# EUROPEAN PATENT APPLICATION

(11) **EP 4 481 022 A1**
(43) Date of publication of application: **25.12.2024**
(21) Application number: 23180496.4
(22) Date of filing: 21.06.2023
(51) Int. Cl.: C11B 9/00, C11D 3/50, A61K 8/40, A61Q 13/00

(54) **FRAGRANCE INGREDIENTS**

(71) Applicant: Givaudan SA, 1214 Vernier (CH)
(72) Inventor: The designation of the inventor has not yet been filed
(74) Representative: Global Patents

(57) **Abstract**

The invention relates to the use of a compound of formula I as a fragrance ingredient, 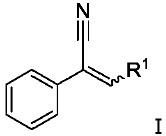 wherein R¹ is a C₆-₇ alkyl group, and to fragrance compositions containing them.

It also relates to a fragrance composition and to a consumer product comprising said compound. A method of manufacturing the fragrance composition is also disclosed.

## Description

### Field of the Invention

The present invention relates to novel uses of compounds of formula I as fragrance ingredients, and to fragrance compositions containing them.

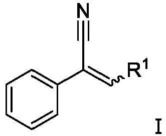

### Background of the Invention

2-phenyl-2-alkene nitriles are known to be useful in perfumery, as described in, for example, WO 2006/060931 and EP 1 637 581. These molecules are considered to have a pleasant, floral, fresh, and Rosacetol-like odour notes and also possess a low odour threshold, in comparison to corresponding saturated compounds.

WO 2008/055372 also describes malodour-counteracting compositions comprising 2-phenyl-2-alkene nitrile and α,β-unsaturated carbonyl compounds.

Nevertheless, to meet consumer demand for sustainable consumer products, there is a need to add to the perfumer's palette of perfumery ingredients, molecules that not only deliver surprising and delightful hedonics, but also deliver on other metrics, such as sustainability and efficacy. There is a particular need for ingredients that not only have a delightful odour, but which are biodegradable and highly impactful.

The inventors have now found in a surprising manner that certain specific 2-phenyl-2-alkene nitrile compounds are not only useful as fragrance ingredients, but are also biodegradable, highly impactful and economical to prepare.

### Detailed Description of the Invention

According to the first aspect of the invention, there is provided the use of a compound of formula I as a fragrance ingredient, 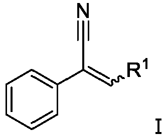 wherein R¹ is a C₆₋₇ alkyl group.

As provided herein, 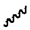 indicates a chemical bond connecting the residue R¹ to the double bond, such that its position relative to the nitrile group is not defined. Accordingly, the skilled person will recognise that compounds of the invention may exist as *E* (*entgegen*) or *Z* (*zusammen*) geometric isomers about the double bond. All geometric isomers of compounds of formula I are included within the scope of the invention.

In particular embodiments of the invention, the compounds of formula I may exist as *Z* (*zusammen*) geometric isomers about the double bond (i.e. the R¹ alkyl group is *cis* to the nitrile group). Thus, particular compounds of the invention that may be mentioned include compounds of formula I-A 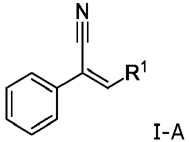 wherein R¹ is a C₆₋₇ alkyl group.

Unless otherwise specified, alkyl groups defined herein may be branched-chain or, preferably, straight-chain. Particular alkyl groups that may be mentioned include hexyl and heptyl.

Thus, in particular embodiments of the invention, R¹ is a straight-chained C₆₋₇ alkyl group (i.e. R¹ is an *n*-hexane or *n*-heptane residue).

In particular embodiments of the invention, the compound of formula I is:

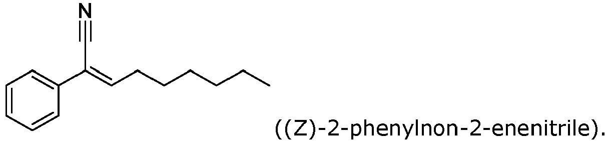

In particular embodiments of the invention, the compound of formula I is:

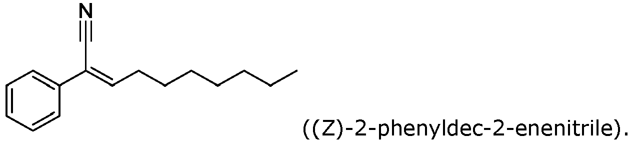

As demonstrated by the examples, the compounds of the invention are useful because they provide pleasant aromas, such as grassy, jasmine, powdery, rosy, floral, fougere and/or hesperidic odour notes. The compounds of the invention are also biodegradable, making their use environmentally friendly. Moreover, they can be prepared at very low cost, making their use in perfumery, even in high concentrations, commercially viable. These characteristics are desirable in consumer applications.

The compounds of the invention show surprising 'hedione-like' character (i.e. having an odour profile which is floral and reminiscent of jasmine), which is uncommon amongst 2-phenyl-2-alkene nitriles. Thus, the fragrance formulations of the invention may show a 'hedione-like' character, even if such formulations contain no hedione.

As indicated herein, the compounds of the invention are useful as fragrance ingredients in the creation of fragrance formulations that are intended to be incorporated into consumer products, such as fine perfumes, household care products, laundry care products, personal care products, and cosmetic products. Thus, in particular embodiments, the use is selected from the group consisting of fine perfumery applications, household care applications, laundry care applications, personal care applications, and cosmetic applications.

The criteria leading to biodegradable molecules is not generally predictable from a consideration of chemical structure. For instance, it is notable that commercial 2-phenyl-2-alkene nitrile derivatives (e.g. Peonile^{™}, Petalia^{®}, Salicynile^{®}) are not biodegradable.

Surprisingly, it has been found that the length of the alkene chain of specific 2-phenyl-2-alkene nitriles determines the biodegradability of the molecules. Specifically, 2-phenyl-2-alkene nitriles with a longer (e.g. eight carbons or more) alkene chain are more biodegradable than corresponding molecules with a shorter (e.g. seven carbons or fewer) alkene chain. As shown in the examples, the compounds of the invention, which feature eight or nine carbon alkene chains (i.e. where R¹ is a C₆₋₇ alkyl group), are biodegradable and also exhibit desirable olfactory performance. In contrast, corresponding compounds featuring alkene chains with seven or fewer carbon atoms have been found not to be biodegradable, and a compound featuring a ten-carbon atom alkene chain was found not to exhibit a good impact.

Olfactory performance may be assessed according to standard procedures known to the person skilled in the art. For example, odour threshold values for volatile perfumery compounds may be determined by a panel of trained individuals using gas chromatography apparatus equipped with a sniff port, where an absolute amount of compound delivered at the sniff port is smelled by each individual. A test compound can be injected consecutively into the apparatus in decreasing concentrations, and the threshold value of a particular compound is determined by the last concentration at which the compound is detectable by a panellist at the retention time corresponding to the compound.

In the context of the present invention, a compound is considered as readily biodegradable if it meets the pass criteria in accordance with an OECD method, referred to as OECD 301F, which is a manometric respirometry test. In this method the pass level for a compound to be considered "ready biodegradability" or "readily biodegradable" is to reach 60 % of theoretical oxygen demand and/or chemical oxygen demand. This pass value has to be reached in a 10-day window within the 28-day period of the test. The 10-day window begins when the degree of biodegradation has reached 10% of theoretical oxygen demand and/or chemical oxygen demand and must end before day 28 of the test. A preferred way of conducting OECD Method 301F is provided herein below.

Given a positive result in a test of readily biodegradable, it may be assumed that a compound will undergo rapid and ultimate biodegradation in the environment (Introduction to the OECD 25 Guidelines for the Testing of Chemicals, Section 3, Part 1: Principles and Strategies Related to the Testing of Degradation of Organic Chemicals; Adopted: July 2003).

An assessment of "inherently biodegradable" can also be made using the OECD Method 301F, although with a different pass criterion. More specifically, the pass criterion is 60 % of theoretical oxygen demand and/or chemical oxygen demand. This pass value can be reached after the 28-day period of the test, which is usually extended to 60 days. No 10-day window applies.

### Formulations

As indicated herein, the compounds of the invention are useful as fragrance ingredients in the formation of fragrance compositions.

Thus, according to a second aspect of the invention, there is provided a fragrance composition comprising a compound of formula I as defined in the first aspect of the invention.

Thus, in particular embodiments, the fragrance composition comprises a compound of formula I and at least one other fragrance ingredient.

The other fragrance ingredients may be any of the ingredients available in the perfumer's palette, such as essential oils, alcohols, aldehydes and ketones, ethers and acetals, esters and lactones, macrocycles and heterocycles, and/or any excipients conventionally used in conjunction with such perfumery ingredients for example, solvents, diluents and other auxiliary agents commonly used in the art.

Particular examples of complementary fragrance ingredients include but are not limited to:
- ethereal oils and extracts, e.g. oak moss absolute, basil oil, tropical fruit oils, such as bergamot oil and mandarin oil, mastic absolute, myrtle oil, palmarosa oil, patchouli oil, petitgrain oil, wormwood oil, lavender oil, rose oil, jasmine oil, ylang-ylang oil and sandalwood oil.
- alcohols, e.g. cis-3-hexenol, cinnamic alcohol, citronellol, Ebanol^{™}, eugenol, farnesol, geraniol, menthol, nerol, rhodinol, Super Muguet^{™}, linalool, phenylethyl alcohol, Sandalore^{™}, terpineol and Timberol^{™} (1 -(2,2,6- Trimethylcyclohexyl)hexanol-3).
- aldehydes and ketones, e.g. citral, hydroxycitronellal, Lilial^{®}, methylnonylacetaldehyde, anisaldehyde, allylionone, verbenone, nootkatone, geranylacetone, α-amylcinnamic aldehyde, Georgywood^{™}, hydroxycitronellal, Iso E Super^{®}, Isoraldeine^{®} (methylionone), Hedione^{®}, maltol, methyl cedryl ketone, and vanillin.
- ether and acetals, e.g. Ambrox^{™}, geranyl methyl ether, rose oxide and Spirambrene^{™}.
- esters and lactones, e.g. benzyl acetate, cedryl acetate, γ-decalactone, Helvetolide^{®}, γ-undecalactone, vetivenyl acetate, cinnamyl propionate, citronellyl acetate, decyl acetate, dimethylbenzylcarbinyl acetate, ethyl acetoacetate, ethyl acetylacetate, cis-3-hexenyl isobutyrate, linalyl acetate and geranyl acetate.
- macrocycles, e.g. ambrettolide, ethylene brassylate and Exaltolide^{®}.
- heterocycles, e.g. isobutylchinoline.

As indicated above, the compounds of the invention show surprising 'hedione-like' character, and as such the compounds of the present invention may find utility as a replacement, either partially or wholly, for hedione.

Accordingly, in particular embodiments of the invention there is provided a fragrance composition comprising a compound of formula (I) that is free or substantially free of hedione.

As indicated herein, the compounds of the invention are particularly useful at least because they are biodegradable. They may be used in fragrance compositions to increase the percentage of biodegradable ingredients contained in such compositions, or they may be used in combination with other biodegradable perfume ingredients to form entirely biodegradable fragrance compositions. Still further, consumer products comprising the fragrance compositions or the compounds of the invention therefore may also be partially or wholly biodegradable.

The fragrance compositions of the present invention may be used in the form of freeoils, or they may be encapsulated, for example with polymers, microcapsules, film formers, absorbents such as carbon or zeolites, cyclic oligosaccharides and mixtures thereof, or the compounds may be chemically bonded to substrates, which are adapted to release the compounds upon application of an external stimulus such as light, enzymes, heat, oxygen, or the like.

Generally, compounds of the invention or the fragrance compositions may be used in consumer products, and consumer products comprising said compounds or compositions represent an additional aspect of the invention.

In particular embodiments of the invention, the consumer product is selected from the group consisting of fine perfumes, laundry products, household products, personal care products, and cosmetics.

Fine perfumes include, but are not limited to parfum, eau de parfum, eau de toilette, eau de cologne, eau fraiche, and perfume oils.

Laundry products which contain a compound or composition of the invention include, but are not limited to, pre-spotters, detergents, detergent boosters, neutralisers, bleaches, de-stainers, and fabric softeners, scent boosters, and conditioners.

Household products which contain a compound or composition of the invention include, but are not limited to, detergents, surface cleaners including hard surface cleaners and all-purpose cleaners, dishwasher deodorising products, kitchen garbage deodorising products, air fresheners, softeners, bleaches, fabric refreshers, scourers and cat litter.

Personal care products and cosmetics which contain a compound or composition of the invention include, but are not limited to, lotions (e.g. after-shave lotion), shampoos, conditioners, styling sprays, mousses, gels, hair wipes, hair sprays, hair pomades, shower gels and bath salts, hygiene products, deodorants, antiperspirants, vanishing creams, depilatories, talcum powders, catamenials, creams and antiperspirants.

The compounds of the present invention can be used by perfumers in widely varying amounts depending upon such factors as the type of consumer product that needs to be fragranced, and the particular hedonic effect that is desired to be achieved.

The compounds are particularly useful because of their pleasant odour and their transparent character. A compound has a transparent character if it has a pleasant character that is not be perceived as very dominant or polarizing to a person with normal olfactive acuity. As such, the compounds can be employed in fragrance formulations in rather high concentrations, acting as a filler at levels as high as 20 or even 25 wt % in fragrance compositions of widely varying olfactive directions. The compounds can be used particularly efficiently in this way because of their relatively low cost.

In particular embodiments of the invention, the concentration of the compound of formula I is from about 0.001 to about 25% by weight, preferably from about 0.001 to about 20% by weight, even more preferably from about 0.001 to about 10% by weight of a fragrance composition, or any sub-range within the ranges referred to above.

However, these values are given only by way of example, since the experienced perfumer may also achieve effects or may create novel accords with lower or higher concentrations.

The term "about" as used herein when referring to a measurable value such as an amount of a compound, dose, time, temperature, and the like, refers to variations of 20%, 10%, 5%, 1%, 0.5%, or even 0.1% of the specified amount. It is contemplated that, at each instance, such terms may be replaced with the notation "±10%", or the like (or by indicating a variance of a specific amount calculated based on the relevant value). It is also contemplated that, at each instance, such terms may be deleted.

### Preparation

Compounds of formula I may be obtained by analogy with the processes known in the literature, or by conventional synthetic procedures, in accordance with standard techniques, from available starting materials using appropriate reagents and reaction conditions. In this respect, the skilled person may refer to inter alia "Comprehensive Organic Synthesis" by B. M. Trost and I. Fleming, Pergamon Press, 1991.

In particular, the compounds of the invention may be prepared in accordance with the techniques described in J. Org. Chem. 2021, 86, 15413-15422.

For example, compounds of the invention may be prepared by a process comprising a reaction of a compound of formula II, 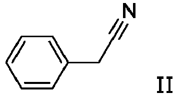 with a compound of formula III, 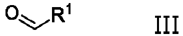 wherein R¹ is as described herein, in the presence of a suitable based (e.g. sodium methoxide and a suitable solvent (e.g. methanol).

Compounds of formula II and formula III may be commercially available, known in the literature, or may be obtained by conventional synthetic procedures, in accordance with standard techniques, from available starting materials using appropriate reagents and reaction conditions.

Accordingly, the compounds of the invention may be prepared at very low cost. Without being bound by theory, the compounds of the invention may therefore enable the provision of known, pleasant odours in an economical manner while also being environmentally friendly, due to the compounds' aforementioned biodegradable properties.

### EXAMPLES

The present invention is explained in greater detail in the following non-limiting examples.

The reaction schemes described below are intended to provide a general description of the methodology employed in the preparation of the compounds of the invention. The examples provided herein are offered to illustrate but not limit the compounds of the invention, as well as the preparation of such compounds and intermediates.

### Abbreviations

- CDCl₃:: Deuterochloroform
- d:: Doublet
- DPG:: Dipropylene glycol
- EI:: Electron ionisation
- equiv:: Equivalents
- GC:: Gas chromatography
- h:: Hour(s)
- HDLD:: High density liquid detergent
- J:: Coupling constant
- m:: Multiplet
- MTBE:: Methyl tert-butyl ether
- q:: Quartet
- quin:: Quintet
- s:: Singlet
- t:: Triplet

### Analytical Methods

GC-MS parameters: Column properties: Trajan (SGE) Capillary GC Column BPX5, 0.22 mm ID x 0.25 µm film thickness x 12 m length, 5% phenyl-95% methylpolysiloxane; Carrier gas: Helium at 1 ml/min constant flow; Temperature program: starting at 50 °C, hold for 2 min, then with 20 °C/min to 240 °C, 35 °C/min to 300 °C, hold for 3 min; Injector temperature: 230 °C; Injector method: split (1:50) injection of 1 µL; Detector: Quadrupole Mass Spectrometer (MS), mass range used m/z 25-450. ¹H- and ¹³C-NMR spectra are referenced to 7.16 ppm and 128.0 ppm, respectively.

### Synthesis Examples

### Example 1: 2-phenylnon-2-enenitrile

A flask was charged with a solution of heptanal (2.0 g, 18 mmol, 1.0 equiv) and 2-phenylacetonitrile (2.10g, 18 mmol, 1.0 equiv) in MeOH (30 ml). NaOMe in MeOH (1.1 equiv. 25% in 4.4 ml, 19 mmol) was added dropwise at 0°C and the mixture was then stirred at room temperature overnight. The mixture was poured on ice water, extracted with MTBE, washed with water and brine, dried over MgSO₄, filtered and concentrated. The crude product was purified twice by chromatography (50g Sfär, Biotage^{®}) to afford 2-phenylnon-2-enenitrile (1.6g, 38%, >95% Z isomer) as a pale-yellow oil.

¹H NMR (400 MHz, CDCl₃) δ: 7.49 - 7.56 (m, 2H), 7.30 - 7.42 (m, 3H), 6.83 (t, J=7.8 Hz, 1H), 2.58 (q, J=7.5 Hz, 2H), 1.49 - 1.60 (m, 2H), 1.23 - 1.45 (m, 6H), 0.90 (t, J=7.1 Hz, 3H) ppm.

¹³C NMR (100 MHz, CDCl₃) δ: 147.2 (d), 133.3 (s), 128.9 (2d), 128.7 (d), 125.5 (2d), 116.6 (s), 115.8 (s), 32.2 (t), 31.5 (t), 28.8 (t), 28.6 (t), 22.5 (t), 14.0 (q) ppm. MS (EI, 70eV): 213 (8, [M+*]), 184 (5), 170 (4), 143 (14), 129 (100), 115 (23), 103 (11), 43 (9).

Odour description (10% in DPG on paper blotter, 24 h): powdery, rosy, Peonile^{™}, grassy, jasmine.

### Example 2: 2-phenyldec-2-enenitrile

A flask was charged with a solution of octanal (2.0g, 15 mmol, 1.0 equiv) and 2-phenylacetonitrile (1.80g, 15 mmol, 1.0 equiv) in MeOH (30 ml). NaOMe in MeOH (1.1 equiv. 25% in 3.9 ml, 1.1 equiv) was added dropwise at 0°C and the mixture was then stirred at room temperature overnight. The mixture was poured on ice water, extracted with MTBE, washed with water and brine, dried over MgSO₄, filtered and concentrated. The crude product was purified by chromatography (50g Sfär, Biotage^{®}) to yield 2-phenyldec-2-enenitrile (2.3g, 63%, >95% Z isomer) as a pale-yellow oil.

¹H NMR (400 MHz, CDCl₃) δ: 7.49 - 7.56 (m, 2H), 7.29 - 7.41 (m, 3H), 6.82 (t, J=7.8 Hz, 1H), 2.58 (q, J=7.6 Hz, 2H), 1.50 - 1.61 (m, 2H), 1.21 - 1.44 (m, 8H), 0.89 (t, J=7.0 Hz, 3H) ppm.

¹³C NMR (100 MHz, CDCl₃) δ: 147.2 (d), 133.2 (s), 128.8 (2d), 128.7 (d), 125.5 (2d), 116.6 (s), 115.8 (s), 32.1 (t), 31.6 (t), 29.1 (t), 29.0 (t), 28.6 (t), 22.5 (t), 14.0 (q) ppm.

MS (EI, 70eV): 227 (9, [M+*]), 198 (6), 184 (5), 156 (7), 143 (17), 129 (100), 115 (33), 103 (13), 77 (4), 43 (18).

Odour description (10% in DPG on paper blotter, 24 h): green, grassy, jasmine, powdery, sweet, Peonile^{™}-like.

### Reference Example 1: 2-phenylpent-2-enenitrile

The compound was obtained from propionaldehyde (2.0 g, 33 mmol, 1.0 equiv), 2-phenylacetonitrile (3.9 g, 33 mmol, 1.0 equiv) and 25% NaOMe in MeOH (8.4 mL, 37 mmol, 1.1 equiv) according to the procedure of Example 1 to afford the title compound as a colourless liquid (2.0 g, 38% yield, mixture of Z/E isomers 97:3).

¹H NMR (400 MHz, CDCl₃, δ/ppm): 7.56-7.51 (m, 2H), 7.43 - 7.32 (m, 3H), 6.82 (t, J = 7.7 Hz, 1H), 2.60 (quin, J = 7.6 Hz, 2H), 1.18 (t, J = 7.5 Hz, 3H).

¹³C NMR (101 MHz, CDCl₃, δ/ppm): 148.3, 133.1, 128.8, 128.7, 125.5, 116.4, 115.3, 25.5, 13.1.

Odour description (10% solution in DPG on paper blotter, 24 h): green, metallic, rose oxyde, leathery, phenolic, thymol, floral, powdery, rosy, benzophenone.

### Reference Example 2: 2-phenylhex-2-enenitrile

2-phenylhex-2-enenitrile was prepared according to a literature procedure (Saudan, L. *et al.,* EP 1 637 581 A1) to give the title compound as a colourless liquid (1.87 g, >95% Z isomer).

¹H NMR (400 MHz, CDCl₃, δ/ppm): 1H NMR (400 MHz, CDCl3, 298 K) Shift (ppm) = 7.55 - 7.52 (m, 2H), 7.42 - 7.32 (m, 3H), 6.84 (t, J = 7.8 Hz, 1H), 2.57 (q, J = 7.6 Hz, 2H), 1.66 - 1.55 (m, 2H), 1.03 (t, J = 7.3 Hz, 3H).

¹³C NMR (101 MHz, CDCl₃, δ/ppm): 146.9, 133.2, 128.8, 128.7, 125.5, 116.6, 116.0, 34.0, 21.9, 13.6.

Odour description (10% solution in DPG on paper blotter): green, mushroom, woody, guaiac wood.

### Reference Example 3: 2-phenylhept-2-enenitrile

The compound was obtained from pentanal (2.84 g, 33 mmol, 1.0 equiv), 2-phenylacetonitrile (3.9 g, 33 mmol, 1.0 equiv) and 25% NaOMe in MeOH (8.4 mL, 37 mmol, 1.1 equiv) according to the procedure of Example 1 to afford the title compound as a colourless liquid (1.7 g, 28% yield, >95% Z isomer).

¹H NMR (400 MHz, CDCl₃, δ/ppm): 7.55 - 7.51 (m, 2H), 7.42 - 7.32 (m, 3H), 6.83 (t, J = 7.8 Hz, 1H), 2.60 (q, J = 7.5 Hz, 2H), 1.61 - 1.52 (m, 2H), 1.48 - 1.40 (m, 2H), 0.97 (t, J = 7.2 Hz, 3H).

¹³C NMR (101 MHz, CDCl₃, δ/ppm): 147.2, 133.3, 128.9, 128.8, 125.5, 116.6, 115.8, 31.9, 30.7, 22.3, 13.8.

Odour description (10% solution in DPG on paper blotter, 24 h): green, salicylate, floral, acetal, peony, rosy.

### Reference Example 4: 2-phenyloct-2-enenitrile

The compound was obtained from hexanal (2.0 g, 20 mmol, 1.0 equiv), 2-phenylacetonitrile (2.3 g, 20 mmol, 1.0 equiv) and 25% NaOMe in MeOH (5.0 mL, 37 mmol, 1.1 equiv) according to the procedure of Example 1 to afford the title compound as a colourless liquid (1.2 g, 30% yield, >95% Z isomer).

¹H NMR (400 MHz, CDCl₃, δ/ppm): 7.55 - 7.52 (m, 2H), 7.42 - 7.32 (m, 3H), 6.84 (t, J = 7.7 Hz, 1H), 2.59 (q, J = 7.6 Hz, 2H), 1.61 - 1.53 (m, 2H), 1.43 - 1.33 (m, 4H), 0.96 - 0.90 (m, 3H).

¹³C NMR (101 MHz, CDCl₃, δ/ppm): 147.2, 133.2, 128.8, 128.7, 125.5, 116.6, 115.8, 32.1, 31.3, 28.3, 22.4, 13.9.

Odour description (10% solution in DPG on paper blotter, 24 h): green, peony, rubbery, oily, fatty, floral.

### Reference Example 5: 2-phenylundec-2-enenitrile

The compound was obtained from nonanal (2.8 g, 20 mmol, 1.0 equiv), 2-phenylacetonitrile (2.3 g, 20 mmol, 1.0 equiv) and 25% NaOMe in MeOH (5.0 mL, 37 mmol, 1.1 equiv) according to the procedure of example 1 to afford the title compound as a colourless liquid (2.2 g, 46% yield, >95% Z isomer).

¹H NMR (400 MHz, CDCl₃, δ/ppm): 1H NMR (400 MHz, CDCl3, 298 K) Shift (ppm) = 7.55 - 7.52 (m, 2H), 7.42 - 7.32 (m, 3H), 6.83 (t, J = 7.8 Hz, 1H), 2.59 (q, J = 7.7 Hz, 2H), 1.61 - 1.53 (m, 2H), 1.44 - 1.22 (m, 10H), 0.92 - 0.87 (m, 3H).

¹³C NMR (101 MHz, CDCl₃, δ/ppm): 147.2, 133.3, 128.8, 128.7, 125.5, 116.6, 115.8, 32.2, 31.8, 29.3, 29.1, 29.1, 28.6, 22.6, 14.0.

Odour description (10% solution in DPG on paper blotter, 24 h): powdery, green, cyclohexyl salicylate, fatty, floral, aldehyde alpha hexyl cinnamic.

### Test Examples

### Test Example 1: Perfume Application Example

Test Example 1 is intended to represent how a skilled perfumer may apply a fragrance formulation to a fabric liquid detergent.

Fragrance formulations comprising either compound Example 1 or compound Example 2 or a reference compound (dipropylene glycol) were prepared in as set out in Table 1 below. These compositions were applied at 0.6% in a high density liquid detergents (HDLD), which was an unperfumed commercial product from Dalli.

**Table 1: Details of tested fragrance formulations**

| **Ingredients** | **Weight parts** | | |
|---|---|---|---|
| | **Reference** | **Formulation A** | **Formulation B** |
| ALDEHYDE C 12 MNA PURE | 25 | 25 | 25 |
| AMBERMAX 10%/TEC | 3 | 3 | 3 |
| AMBROFIX | 5 | 5 | 5 |
| BENZYL ACETONE | 15 | 15 | 15 |
| COUMARIN PURE CRYSTALS | 5 | 5 | 5 |
| DAMASCONE DELTA | 3 | 3 | 3 |
| DIHYDRO MYRCENOL | 180 | 180 | 180 |
| DIPROPYLENE GLYCOL | 20.7 | 20.7 | 20.7 |
| GALBANONE 10 | 15 | 15 | 15 |
| GERANIOL INTERMEDIATE 60 | 60 | 60 | 60 |
| HEDIONE | 80 | 80 | 80 |
| HEXYL SALICYLATE | 100 | 100 | 100 |
| ISO E SUPER | 100 | 100 | 100 |
| ISOEUGENOL | 5 | 5 | 5 |
| JAVANOL | 0.8 | 0.8 | 0.8 |
| LAVANDIN GROSSO OIL FRANCE ORPUR | 20 | 20 | 20 |
| METHYL SALICYLATE | 1.5 | 1.5 | 1.5 |
| NYMPHEAL | 30 | 30 | 30 |
| PEONILE | 60 | 60 | 60 |
| PHENYL ETHYL ACETATE | 60 | 60 | 60 |
| ROSE OXIDE CO | 3 | 3 | 3 |
| SERENOLIDE | 70 | 70 | 70 |
| TRICYCLAL | 8 | 8 | 8 |
| UNDECAVERTOL | 20 | 20 | 20 |
| VELVIONE | 10 | 10 | 10 |
| DIPROPYLENE GLYCOL | 100 | 0 | 0 |
| (Z)-2-PHENYLNON-2-ENENITRILE Example 1 | 0 | 100 | 0 |
| (Z)-2-PHENYLDEC-2-ENENITRILE Example 2 | 0 | 0 | 100 |
| **Total:** | 1000 | 1000 | 1000 |

Substitution of 100 weight parts of dipropylene glycol in Formulation A by 100 weight parts of (Z)-2-phenylnon-2-enenitrile was found to add a more floral (jasmine, rosy) and green (leaf, vegetal) accent to the accord.

Substitution of 100 weight parts of dipropylene glycol in Formulation B by 100 weight parts of (Z)-2-phenyldec-2-enenitrile was found to add a more floral (jasmine, rosy) and powdery accent to the accord.

When compared to the reference formulation, both Formulation A and Formulation B deliver a similar benefit to performance and longevity on dry fabric, and both formulations benefit the overall floral odour and roundness of the accord on dry fabric.

### Test Example 2: GC Odour Threshold Measurements

The detection odour threshold is defined as being the smallest concentration of an odour that is perceptible by a statistically significant number of panellists and is calculated as the geometric mean of the detection odour threshold values of different panellists.

The detection odour threshold can be determined by standard olfactometric or standard gas-chromatographic methods known to the person skilled in the art. In this example they were measured by a standard gas-chromatographic (GC) method: solutions of the respective test compounds were injected at decreasing concentrations into a GC instrument equipped with a sniff port. The respective test compounds were injected until a panellist fails to detect the substance at the sniffing port. The last concentration of a compound detected at the correct retention time is the individual detection odour threshold (Neuner-Jehle, N., Etzweiler, F. (1994). The Measuring of Odors. In: Müller, P.M., Lamparsky, D. (eds) Perfumes. Springer, Dordrecht. https://doi.org/10.1007/978-94-011-3826-0 6).

The results are listed in Table 2.

**Table 2: Details of GC odour threshold measurements for tested compounds**

| **Compound** | | **GC Odour threshold** |
|---|---|---|
| **Example 1** | 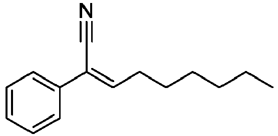 | 0.25 ng |
| (2-phenylnon-2-enenitrile) | | |
| **Example 2** | 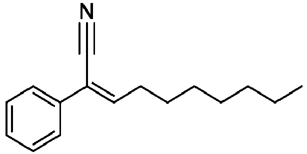 | 0.34 ng |
| (2-phenyldec-2-enenitrile) | | |
| **Reference Example 1** | 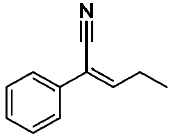 | 12.0 ng |
| (2-phenylpent-2-enenitrile) | | |
| **Reference Example 2** | 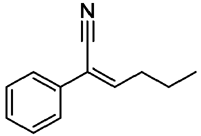 | 0.53 ng |
| (2-phenylhex-2-enenitrile) | | |
| **Reference Example 3** | 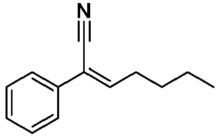 | 0.04 ng |
| (2-phenylhept-2-enenitrile) | | |
| **Reference Example 4** | 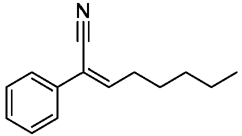 | 2.5 ng |
| (2-phenyloct-2-enenitrile) | | |
| **Reference Example 5** | 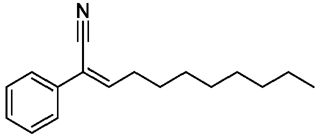 | 7.3 ng |
| (2-phenylundec-2-enenitrile) | | |

The results show that compounds of the present invention (Examples 1 and 2) have a GC odour detection threshold of less than 1 ng, which is indicative of good performance in perfume applications.

Reference compounds 2 and 3 also have a GC odour detection threshold of below 1 ng.

In contrast, several comparative compounds with a shorter (Reference Examples 1 and 4) or a longer alkyl side chain (Reference Example 5) have a significantly higher (greater than 5 times) GC odour detection threshold in comparison to the compounds of the present invention (Examples 1 and 2).

### Test Example 3: Biodegradability Assessments

The biodegradability assessment of the test compounds was performed in accordance with the OECD Guidelines for the Testing of Chemicals No. 301F.

A measured volume of inoculated mineral medium, containing a known concentration of test compound as the nominal sole source of organic carbon, was stirred in a closed flask at a constant temperature (22 ± 1°C) for up to 60 days. The consumption of oxygen was determined by measuring the pressure drop in a respirometer flask. The biodegradation in % terms is the Biological Oxygen Demand (BOD), i.e. amount of oxygen taken up by the microbial population during biodegradation of the test compound, corrected for uptake by blank inoculum, as a percentage of the Theoretical Oxygen Demand (ThOD), calculated from the elemental composition.

Fresh activated sludge from a biological waste water treatment plant treating predominantly domestic sewage was used. The sludge was collected in the morning, washed three times in the mineral medium and kept under aerobic conditions until being used on the same day.

Test substance samples were weighed and added directly to the test flasks of the respirometers at a concentration of 30.0 mg/L in 255 mL of test medium. For reference substance samples, 12.75 mg (corresponding to 50.0 mg/L in 255 mL of test medium) were weighed in small aluminium boats and added directly to the test flasks of the respirometers. Flasks were filled with 250 mL of mineral medium. Then 5.00 mL of suspended sludge diluted to a concentration of 1.53 g/L dry matter was added. Every day the oxygen consumption of each flask was recorded and used for the calculation of biodegradation.

The results of biodegradability assessment under OECD Guideline No. 301F are summarized in Table 3.

**Table 3: Details of biodegradability assessments for tested compounds**

| **Compound** | | **Biodegradation** | **Result** |
|---|---|---|---|
| **Example 1** | 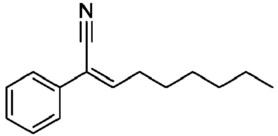 | 76% | Inherently biodegradable |
| **Example 2** | 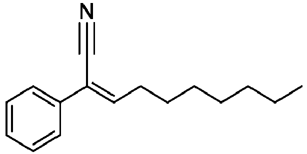 | 89% | Inherently biodegradable |
| **Reference Example 1** | 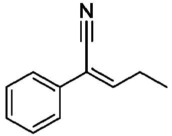 | 0% | Not biodegradable in the conditions of the test |
| **Reference Example 2** | 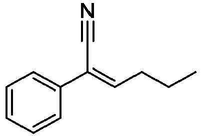 | 0% | Not biodegradable in the conditions of the test |
| **Reference Example 3** | 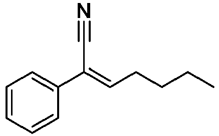 | 13% | Not biodegradable in the conditions of the test |
| **Reference Example 4** | 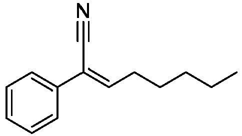 | 25% | Not biodegradable in the conditions of the test |
| **Reference Example 5** | 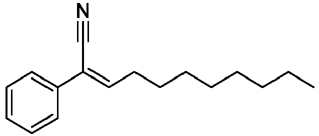 | 95% | Readily biodegradable |

The results show that compounds of the present invention (Examples 1 and 2) are inherently biodegradable.

A compound can be classified as readily biodegradable, if it reaches the pass level of 60% biodegradation within a 28-day period. The 10-day window criterion must be met (which begins when the degree of biodegradation has reached 10% and 60% biodegradation has to be reached within 10 days of the beginning of the test and before day 28 of the test).

If the pass level criterion is met within a 28-day period, but not the 10-day window criterion, a compound can be regarded as inherently biodegradable. A compound can also be considered as inherently biodegradable, if, in a prolonged OECD Guideline No. 301F test, the pass level of 60% biodegradation is not reached after 28 days, but before, or on, day 60 of the test.

Reference Examples 1 to 4 were both found to be not inherently biodegradable.

### Conclusions to Test Examples 2 and 3

Only the compounds of the present invention (Examples 1 and 2) met both criteria of having a good GC odour threshold (Test Example 2) and being (inherently) biodegradable (Test Example 3).

## Claims

1. Use of a compound of formula I as a fragrance ingredient, 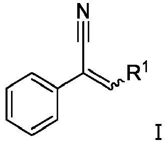 wherein R¹ is a C₆₋₇ alkyl group.

2. A use according to Claim 1, wherein the compound is:

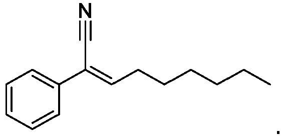

3. A use according to Claim 1, wherein the compound is:

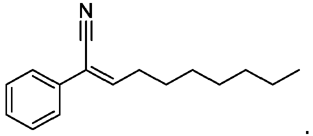

4. A use according to anyone of Claims 1 to 3, wherein the use is selected from the group consisting of perfume applications, household applications, laundry applications, personal care applications, and cosmetic applications.

5. A fragrance composition comprising a compound of formula I as defined in any one of Claims 1 to 3.

6. A consumer product comprising a compound of formula I as defined in any one of Claims 1 to 3 or a fragrance composition as defined in Claim 5.

7. A consumer product according to Claim 6, wherein the consumer product is selected from the group consisting of a fine perfume, a laundry product, a household product, a personal care product, and a cosmetic.

8. A consumer product according to Claim 6 or Claim 7, wherein the consumer product is biodegradable.

9. A consumer product according to any one of Claims 6 to 8, wherein the consumer product is a biodegradable perfume.

10. A consumer product according to any one of Claim 6 to Claim 9, wherein the concentration of the compound of formula (I) is up to about 25% by weight of the consumer product.

11. A method of manufacturing a fragrance composition as defined in Claim 5, comprising the step of incorporating a compound of formula I as defined in Claims 1 to 3 into one or more base materials.

12. A method according to Claim 11 wherein the fragrance composition is selected from the group consisting of perfumes, household products, laundry products, personal care products, and cosmetics.
